# EUROPEAN PATENT SPECIFICATION

(11) **EP 0 786 243 B1**
(45) Date of publication and mention of the grant of the patent: **13.11.2002**
(21) Application number: 96309120.2
(22) Date of filing: 13.12.1996
(51) Int. Cl.: A61F 13/62

(54) **Disposable diaper**
Wegwerfwindel
Couche culotte jetable

(30) Priority: 23.01.1996 JP 900396
(43) Date of publication of application: 30.07.1997
(73) Proprietor: UNI-CHARM CORPORATION, Kawanoe-shi Ehime-ken (JP)
(72) Inventor: Matsushita, Michiyo, Iyomishima-shi, Ehime-ken (JP)
(74) Representative: Parry, Christopher Stephen

(56) References cited:
- WO-A-90/07313
- GB-A- 2 284 742
- US-A- 5 049 145
- US-A- 5 137 526

## Description

The present invention relates to a disposable diaper.

As a fastener for a garment of this type, what is called a tape fastener has conventionally been used, which comprises a combination of a hook member and a loop member and is commonly known under the trade name 'Velcro Tape' or 'Magic Tape'. With this fastener hooks and loops of both members extending substantially in vertical direction are brought into a mechanical engagement as the hook member and the loop member are put one upon another.

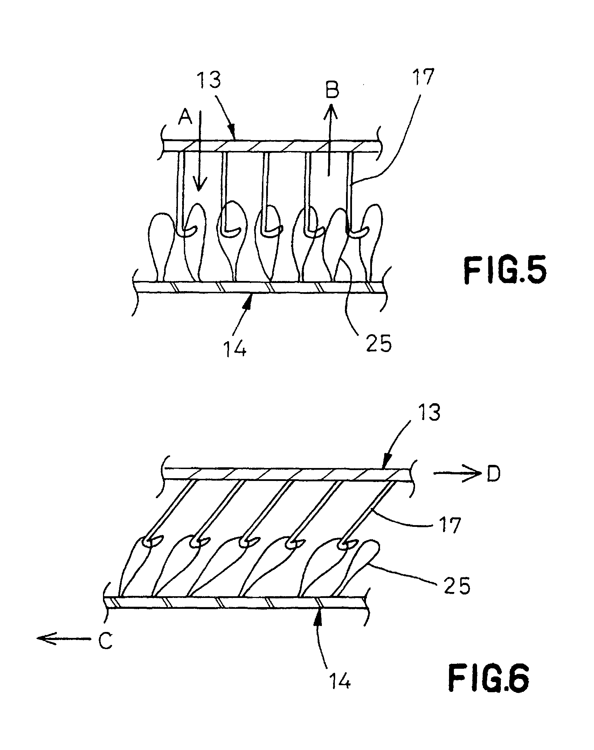

Figs. 5 and 6 of the accompanying drawings are side views illustrating a manner in which hooks 17 on a hook member 13 are engaged with loops 25 on a loop member 14. Referring to Fig. 5, the hook member 13 is engaged with the loop member 14 as the hook member 13 is put on the loop member 14 in a direction as indicated by an arrow A and released therefrom as the hook member 13 is pulled vertically as indicated by an arrow B. Referring now to Fig. 6, these two members 13, 14 are tensioned, when they are pulled in horizontally opposite directions as indicated by arrows C, D from their mutually engaged states as illustrated by Fig. 5. Fig. 6 reveals that many of the hooks 17 are positively brought into engagement with the corresponding loops 25. In the state illustrated by Fig. 5 on the other hand, the hooks 17 are merely inserted into spaces defined among the loops 25 and only a few of the hooks 17 are in engagement with the corresponding loops 25. As a consequence, the hook member 13 can be easily released from the loop member 14 as the hook member 13 are pulled upward from the loop members 14.

With respect to such a fastener, Japanese Laid-Open Patent Application No. Hei4-276251 discloses that a loop member may be intermittently bonded to a garment to facilitate many of hooks to be caught by corresponding loops as a hook member is horizontally pulled.

With a garment put on a wearer and fastened by engaging a hook member with a loop member of a fastener, the hook member is sometimes released from the loop member due to a vertically oriented force tending to release the hook member from the loop member carelessly exerted on the fastener. To avoid such a situation, there is always a demand for a fastener so improved that, with a garment put on a wearer, a mutual engagement of these two members can not be easily released simply by an unintentional movement of the hook member in the direction vertical to the loop member but released only by positively pulling the hook member in the vertical direction when it is desired to take off the garment from the wearer. While the previously mentioned Laid-Open Patent Application No. Hei4-276251 discloses a means ensuring a hook member and a loop member to be engaged with each other as the hook member is moved in a horizontal direction relatively to the loop member, none of means is disclosed, by which an engagement of these two members can be ensured as the hook member is moved in the vertical direction relative to the loop member.

WO-A-90/07313 describes a washable cotton diaper having wings on the rear portion which attach to the front portion by means of hook and loop elements. The hook member on each wing is sewn to the wing over only a part of its length such that the outer section of the hook member is left unattached.

In view of problems as have been described above, it is a principal object of the invention to provide a fastener used for a garment improved so as to ensure that a hook member should not be easily released from a loop member even if the hook member is moved in a vertical direction away with respect to the loop member.

The object set forth above is achieved, according to the invention, by a disposable diaper comprising: a liquid permeable top sheet, a liquid impermeable back sheet and a liquid absorbent core disposed between the top sheet and the back sheet; the diaper comprising a front body area, a rear body area and a crotch area extending between the front and rear body areas; and a tape fastener for the diaper comprising a hook member and a loop member releasably engageable with each other; wherein the loop member is supported on the back sheet in the front body area of the diaper; and wherein the hook member is supported on a tape member having an inner end and a free outer end and being connected at its inner end to the back sheet in the rear body area of the diaper and extending laterally outwardly thereof, the hook member comprising a base sheet having a top surface and a back surface and a plurality of hooks extending vertically from the top surface of the base sheet, wherein the back surface of the base sheet comprises a bonded region (20) and a non-bonded region with respect to the tape member, the non-bonded region extending over the full width of the base sheet transversely of the length of the tape member, and some of the hooks vertically extend from the top surface corresponding to the non-bonded region; the non-bonded region being located towards the free outer end of the tape member which provides a grip zone to be held by fingers to release the fastener; the number of hooks in the non-bonded region being smaller than the number of hooks in the bonded region; and 2-20 pieces of the hooks (17) per 5mm length are arranged on the base sheet (16) longitudinally as well as transversely thereof and the outer region (18) is provided with the hooks (17) arranged in 2-20 rows each extending transversely thereof.

Embodiments of the present invention are described below with reference to the accompanying drawings, in which:
Fig. 1 is a perspective view showing a disposable diaper;
Fig. 2 is a fragmentary perspective view showing the fastener of the diaper as partially broken away as viewed in a direction as indicated by arrows from a section taken along line II-II in Fig. 1.
Fig. 3 is a side view showing a hook member and a loop member as they have been engaged with each other;
Fig. 4 is a side view showing the hook member as it is being released from the loop member;
Fig. 5 is a fragmentary side view showing the hook member and the loop member as they have been engaged with each other; and
Fig. 6 is a fragmentary side view showing the hook member and the loop member as they are being pulled from their positions in Fig. 5 in horizontally opposite directions.

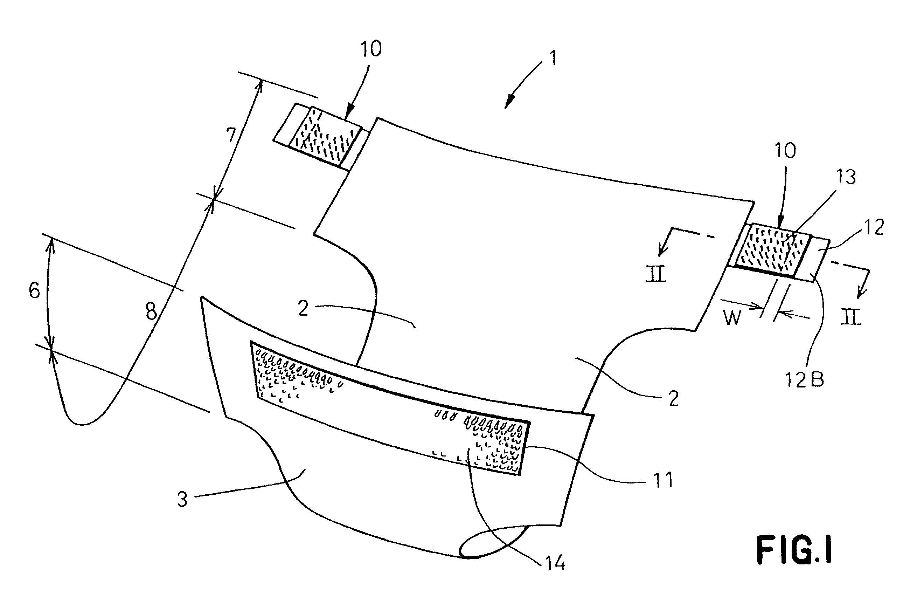

A disposable diaper 1 shown by Fig. 1 in a perspective view comprises a liquid-permeable topsheet 2, a liquid-impermeable backsheet 3 and a liquid-absorbent core (not shown) disposed between these two sheets 2, 3 and longitudinally composed of a front body area 6, a rear body area 7 and a crotch area 8 extending between these two areas 6, 7. A fastener for the diaper comprises a fastening tape 10 and a target tape 11. A pair of the fastening tapes 10 respectively extends from transversely opposite side edges of the rear body area 7, and the target tape 11 is bonded to an outer surface of the backsheet 3 and circumferentially extends. Each of the fastening tapes 10 comprises a tape member 12 and a hook member 13 bonded to the top surface of the tape member 12. The target tape 11 has a loop member 14. A combination of these hook member 13 and loop member 14 is commonly known under the trade name of 'Velcro Tape' or 'Magic Tape' and both members 13, 14 cooperate with each other so that they are detachably fastened together and thereby reliably hold a diaper 1 on a wearer as the hook member 13 is forced against the loop member 14. To take off the diaper 1 from the body, an outer end 12B of the tape member 12 as will be described later is held by fingers and the hook member 13 is progressively released from the loop member 14 from the outer end 12B toward the inner end 12A opposed to the outer end 12B.

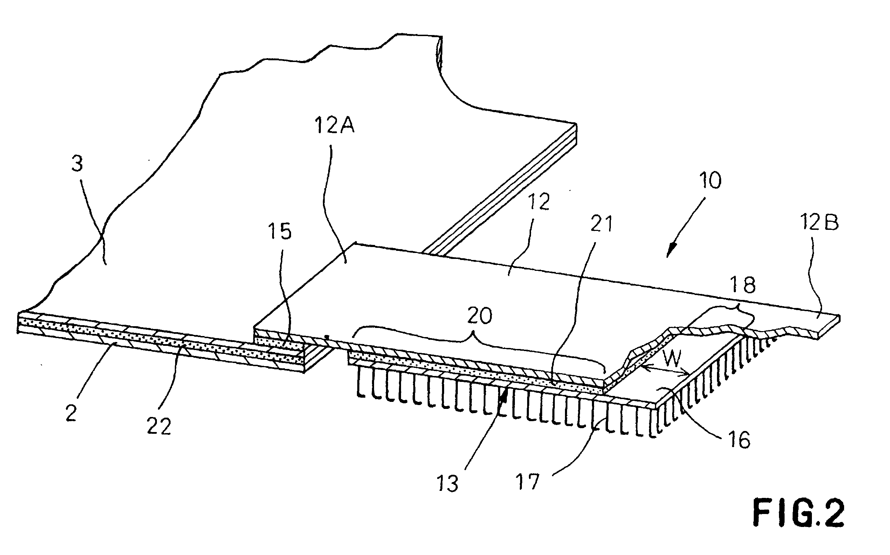

Fig. 2 is a fragmentary perspective view showing the fastener 10 as partially broken away as viewed in the direction as indicated by arrows from a section taken along line II-II in Fig. 1. The inner end 12A of the tape member 12 constituting the fastener 10 is inseparably bonded to the back surface, i.e., outer surface, of the backsheet 3 by means of hot melt type adhesive 15 and the outer end 12B of the tape member 12 serves as a grip zone used to release the fastening tape 10. The hook member 13 bonded to the top surface, i.e., inner surface, of the tape member 12 between the inner and outer ends 12A, 12B comprises a base sheet 16 and hooks 17 extending from the base sheet 16 in vertical direction and the base sheet 16 is inseparably bonded to the tape member 12 over a region 20 thereof except an outer end region 18 defined by a length W and fully extending over a width of the sheet 16 (refer also to Fig. 1) by means of hot melt type adhesive 21. The outer end region 18 defines a non-bonded zone with respect to the tape member 12. 2 - 20 pieces of the hook 17, preferably 5 - 15 pieces of the hook 17 per 5mm length are arranged on the base sheet 16 longitudinally as well as transversely thereof and the outer end region 18 is provided with the hooks 17 arranged in 2 - 20 rows each extending transversely thereof. It should be understood that the top- and backsheets 2, 3 are bonded together by means of hot melt adhesive 22 along transversely opposite side edges of the diaper 1.

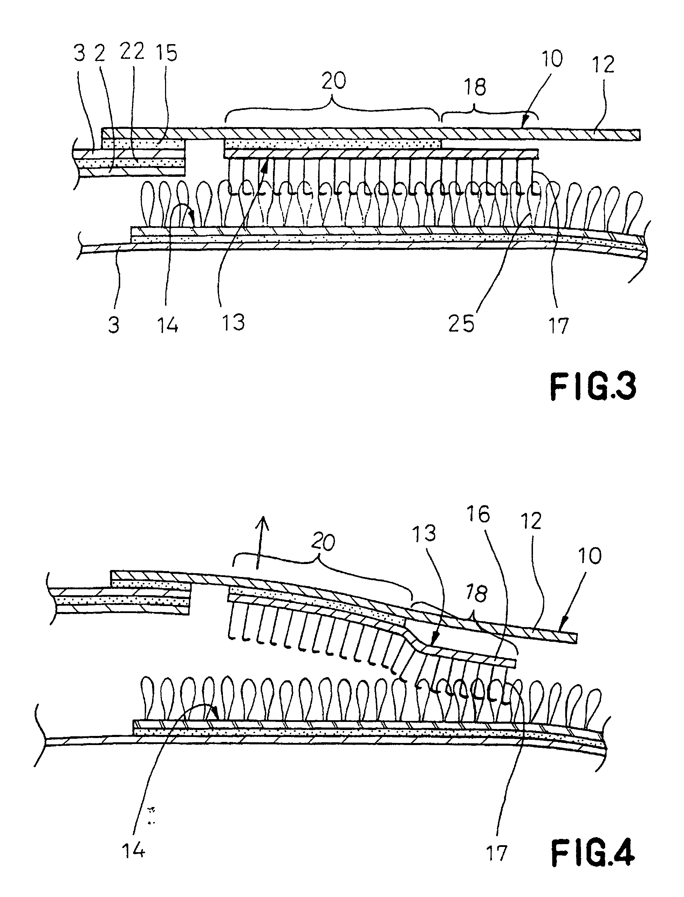

Fig. 3 is a side view similar to Fig. 5, illustrating a state in which the hook member 13 is pressed in the vertical direction against the loop member 14 and brought into engagement with the loop member 14. In this state, a plurality of the vertically oriented hooks 17 are merely inserted into spaces defined among a plurality of the loops 25 which are also oriented in the substantially vertical direction and therefore a relatively small number of the hooks 17 are effectively engaged with the correspondingly small number of the loops 25.

Fig. 4 is a schematic diagram illustrating a state after the fastening tape 10 has moved upward in vertical direction from its position shown by Fig. 3 relatively to the loop member 14. As illustrated, the hooks 17 follow such a movement of the tape fastener 10 and rather smoothly leave the spaces defined among the loops 25 so far as the region 20 of the hook member 13 bonded to the tape member 12 is concerned. In the outer end region 18 (non-bonded region), on the other hand, the movement of the fastening tape 10 causes the hooks 17 to be tilted so as to be laterally brought into engagement with the corresponding loops 25 and thereby the hook member 13 is prevented from being released away from the loop member 14. Obviously, the number of the rows of the hook 17 associated with the outer end region 18 of the base sheet 16 may be selected from a range of 2 - 20 and accordingly the fastening tape 10 can be positively released from the target zone 11 without any difficulty.

In the hook member 13 used with the diaper, the base sheet 16 has a bending resistance less than 0.1g as measured by a Gurley Stiffness Tester. Bonding of the diaper's various components such as the hook member 13 and the loop member 14 may be achieved by utilizing, in addition to adhesive such as hot melt adhesive having appropriate softness, heat-sealing so far as components to be bonded are heat-sealable.

With the diaper of the invention, there is no apprehension that the hook member might be readily and unintentionally released off from the loop member even when the hook member moves in the vertical direction away with respect to the loop member with which the hook member has been engaged, because the outer end region of the hook member is left as the non-bonded region with respect to the base sheet to which the remaining region of the hook member is bonded and the outer end region is provided with 2 - 20 rows of the hooks.

## Claims

1. A disposable diaper comprising:
a liquid permeable top sheet (2), a liquid impermeable back sheet (3) and a liquid absorbent core disposed between the top sheet and the back sheet;
the diaper comprising a front body area (6), a rear body area (7) and a crotch area extending between the front and rear body areas; and
a tape fastener for the diaper comprising a hook member (13) and a loop member (14) releasably engageable with each other;
wherein the loop member (14) is attached to the back sheet (3) in the front body area (6) of the diaper; and
wherein the hook member is carried on a tape member (12) having an inner end (12A) and a free outer end (12B) and being attached at its inner end (12A) to the back sheet (3) in the rear body area (7) of the diaper and extending laterally outwardly thereof, the hook member (13) comprising a base sheet (16) having a top surface and a back surface and a plurality of hooks (17) extending vertically from the top surface of the base sheet, wherein,
the back surface of the base sheet (16) comprises a bonded region (20) and a non-bonded region (18) with respect to the tape member (12), the non-bonded region (18) extending over the full width of the base sheet transversely of the length of the tape member, and some of the hooks (17) vertically extend from the top surface corresponding to the non-bonded region (18);
the non-bonded region (18) being located towards the free outer end (12B) of the tape member (12) which provides a grip zone to be held by fingers to release the fastener;
the number of hooks in the non-bonded region (18) being smaller than the number of hooks in the bonded region (20); and
2-20 pieces of the hooks (17) per 5mm length are arranged on the base sheet (16) longitudinally as well as transversely thereof and the outer region (18) is provided with the hooks (17) arranged in 2-20 rows each extending transversely thereof.

2. The diaper according to Claim 1 or Claim 2, wherein the base sheet (16) having a bending resistance of less than 0.1g as measured by a Gurley Stiffness Tester.

## Patentansprüche

1. Wegwerfwindel, aufweisend:
eine flüssigkeitsdurchlässige Oberschicht (2), eine flüssigkeitsundurchlässige Unterschicht (3) und einen dazwischen angeordneten flüssigkeitsabsorbierenden Kern, wobei die Windel einen Vorderkörperbereich (6), einen Hinterkörperbereich (7) und einen dazwischen verlaufenden Schrittbereich aufweist, und
einen Bandverschluß für die Windel, der ein Hakenelement (13) und ein Schlaufenelement (14) beinhaltet, die lösbar ineinander eingreifen können, wobei
das Schlaufenelement (14) im Vorderkörperbereich (6) der Windel an der Unterschicht (3) angebracht ist,
das Hakenelement auf einem Bandelement (12) getragen ist, das ein Innenende (12A) und ein freies Außenende (12B) aufweist und an seinem Innenende (12A) im Hinterkörperbereich (7) der Windel an der Unterschicht (3) angebracht ist und quer nach außen dazu verläuft, und eine Basisschicht (16) mit einer oberen Fläche und einer Rückenfläche sowie eine Vielzahl von vertikal von der oberen Fläche der Basisschicht verlaufenden Haken (17) aufweist,
die Rückenfläche der Basisschicht (16) mit Bezug zum Bandelement (12) einen angehefteten Abschnitt (20) und einen nicht angehefteten Abschnitt (18) aufweist, wobei der nicht angeheftete Abschnitt (18) über die gesamte Breite der Basisschicht quer zur Länge des Bandelements verläuft und sich einige der Haken (17) vertikal von der dem nicht angehefteten Abschnitt (18) entsprechenden oberen Fläche aus erstrecken,
der nicht angeheftete Abschnitt (18) zum freien Außenende (12B) des Bandelements (12) hin angeordnet ist, das einen Griffbereich zum Halten mit den Fingern beim Öffnen des Verschlusses zur Verfügung stellt,
die Zahl der Haken in dem nicht angehefteten Bereich (18) kleiner als die Zahl in dem angehefteten Bereich (20) ist, und
in Längsrichtung sowie in Querrichtung der Basisschicht (16) 2 bis 20 einzelne Haken (17) pro 5 mm Länge darauf angeordnet sind und die Haken (17) im Außenbereich in 2 bis 20 quer dazu verlaufenden Reihen vorgesehen sind.

2. Windel nach Anspruch 1, wobei die Basisschicht (16), mit einem Gurley Steifigkeits-Meßgerät gemessen, einen Biegewiderstand von weniger als 0,1 g aufweist.

## Revendications

1. Couche jetable comprenant :
une feuille supérieure perméable aux liquides (2), une feuille inférieure imperméable aux liquides (3) et une partie centrale absorbant les liquides disposée entre la feuille supérieure et la feuille inférieure ;
la couche comprenant une région frontale du corps (6), une région arrière du corps (7) et une région d'entre-jambes s'étendant entre les régions frontale et arrière du corps ; et
une bande de fermeture comprenant un élément à crochets (13) et un élément à boucles (14) pouvant être mis en prise de manière libérable l'un avec l'autre ;
dans laquelle l'élément à boucles (14) est fixé à la feuille inférieure (3) au niveau de la région frontale du corps (6) de la couche ; et
dans laquelle l'élément à crochets est fixé sur un élément de bande (12) qui comporte une extrémité interne (12A) et une extrémité externe libre (12B), et qui est attaché au niveau de son extrémité interne (12A) à la feuille inférieure (3) dans la région arrière du corps (7) de la couche et qui s'étend latéralement vers l'extérieur de celle-ci, l'élément à crochets (13) comprenant une feuille de base (16) présentant une surface supérieure et une surface inférieure ainsi qu'une pluralité de crochets (17) s'étendant verticalement à partir de la surface supérieure de la feuille de base, dans lequel,
la surface inférieure de la feuille de base (16) comporte une région collée (20) et une région non collée (18) par rapport à l'élément de bande (12), la région non collée (18) s'étendant par-dessus la pleine largeur de la feuille de base transversalement par rapport à la longueur de l'élément de bande, et certains des crochets (17) s'étendant verticalement à partir de la surface supérieure correspondant à la région non collée (18) ;
la région non collée (18) étant située vers l'extrémité externe libre (12B) de l'élément de bande (12) formant une zone de prise devant être tenue par les doigts pour libérer la fermeture ;
le nombre de crochets dans la région non collée (18) étant inférieur au nombre de crochets dans la région collée (20) ; et
2 à 20 crochets (17) par 5 mm sont disposés sur la feuille de base (16), longitudinalement ainsi que transversalement par rapport à celle-ci, et la région externe (18) est pourvue de crochets (17) disposés en 2 à 20 rangées, chacune s'étendant transversalement par rapport à celle-ci.

2. Couche selon la revendication 1 ou la revendication 2, dans laquelle la feuille de base (16) présente une résistance à la flexion inférieure à 0,1 g mesurée par un testeur de rigidité Gurley.
